# EUROPEAN PATENT SPECIFICATION

(11) **EP 0 804 417 B1**
(45) Date of publication and mention of the grant of the patent: **04.06.2003**
(21) Application number: 96900349.0
(22) Date of filing: 12.01.1996
(51) Int. Cl.: C07D 211/60

(54) **RACEMISATION PROCESS FOR USE IN THE MANUFACTURE OF LEVOBUPIVACAINE AND RELATED PIPERIDINECARBOXANILIDE ANAESTHETIC AGENTS**
RACEMISIERUNGSVERFAHREN ZUR HERSTELLUNG VON LEVOBUPIVACAIN UND VERWANDTE PIPERIDIN CARBOXANILIDE MIT ANÄSTHETISCHER WIRKUNG
PROCEDE DE RACEMISATION UTILISABLE DANS LA FABRICATION DE LA LEVOBUPIVACAINE ET DES AGENTS ANESTHESIQUES CORRESPONDANTS A BASE DE PIPERIDINECARBOXANILIDE

(30) Priority: 18.01.1995 GB 9501071
(43) Date of publication of application: 05.11.1997
(73) Proprietor: Darwin Discovery Limited, Slough Berks SL1 3WE (GB)
(72) Inventor: DYER, Ulrich, Conrad Chiroscience Limited, Cambridge CB4 4WE (GB); LANGSTON, Marianne Chiroscience Limited, Milton Road Cambridge CB4 4WE (GB); WOODS, Martin Chiroscience Limited, Milton Road Cambridge CB4 4WE (GB)
(74) Representative: Perry, Robert Edward
(86) International application number: GB9600067
(87) International publication number: WO96022281

(56) References cited:
- FR-A- 2 301 498
- ACTA PHARMACEUTICA SUECICA, vol. 25, no. 3, 1988 pages 121-132, P. FYHR, C. HOGSTROEM 'A Preformulation Study on the Kinetics of the Racemisation of Ropivacaine Hydrochloride' cited in the application
- J. ORG. CHEM., vol. 48, 1983 pages 843-846, S. YAMADA ET. AL. 'Method for the Racemisation of Optically Active Amino Acids'
- J. ORG. CHEM., vol. 43, no. 1, 6 January 1978 pages 1-5, G.G. SMITH ET. AL. 'Factors Affecting the Rate of Racemisation of Amino Acids and Their Significance to Geochronology'
- BULL. CHEM. SOC. JPN., vol. 64, 1991 pages 3251-3255, T. SHIRAIWA ET. AL. 'Asymmetric Transformation of Proline and 2-Piperidinecarboxylic Acid via Formation of Salts with Optically Active Tartaric Acid'
- CHEM. PHARM. BULL., vol. 18, no. 9, September 1970 pages 1794-1798, M. SATO ET. AL. 'Studies on the Racemisation of Amino Acids and Their Derivatives'

## Description

### Field of the Invention

This invention relates to the racemisation of optically-enriched piperidine-2-carboxanilides. In particular, the process is suitable for use in the manufacture of levobupivacaine and related piperidinecarboxanilide anaesthetic agents.

### Background to the Invention

Compounds of formula 1 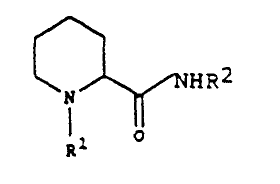 wherein R² is 2,6-dimethylphenyl and R¹ is methyl (mepivacaine), *n*-propyl (ropivacaine as S-enantiomer) or *n*-butyl (bupivacaine) are widely used as local anaesthetics.

Biological studies have shown that the (*S*)-enantiomers of such N-alkyl-piperidine-2-carboxanilides display lower cardiotoxicity than the corresponding racemates, whilst maintaining the same anaesthetic potency, and are therefore more beneficial for clinical uses. Thus there is a requirement for efficient processes to manufacture compounds of formula 1 in the form of single enantiomers. For this purpose, conventional resolution approaches invariably afford up to 50% of the unwanted enantiomer. To improve atom utilisation in such processes, it is desirable to recycle the unwanted enantiomer by effecting its racemisation in order to provide material suitable for subsequent resolution.

Friberger *et al,* Acta. Pharm. Suec. (1971) 8:361-364, report a study of the solubility and partition coefficients of the racemates and enantiomers of mepivacaine and bupivacaine. It is reported that racemic bupivacaine is more soluble than the isomers at a pH above 6. All of the compounds tested have solubilities decreasing to low levels, especially for bupivacaine, at pH values approaching neutrality.

Fyhr *et al*, Acta.Pharm.Suec. (1988) 25:121-132, report the racemisation of optically-enriched ropivacaine hydrochloride in dilute aqueous solution at pH 1-6 and 80-130°C. HCl or citric acid was present, in order to establish the pH. The conclusions of this pre-formulation stability study were that the racemisation involves hydroxyl ion-catalysed racemisation of the *N*-protonated species. This study provides no useful indication as to how to conduct racemisation as such, and does not suggest any volume-efficient commercial process.

### Summary of the Invention

The present invention is based on the surprising discovery that piperidine-2-carboxanilides, including compounds of formula 1 wherein R¹ is methyl, *n*-propyl or *n*-butyl and R² is 2,6-dimethylphenyl, undergo rapid racemisation when heated in aqueous solution, provided that an organic cosolvent is present. The practical nature of this discovery is evident in that much more concentrated systems can be used than in the prior art.

Whereas, at concentrations of 30 mg/ml, at a pH above 5, the use of conditions otherwise specified by Fyhr *et al* lead to complete inhibition of racemisation of ropivacaine and bupivacaine, the rate of racemisation can be increased, under the conditions used in this invention, with increasing pH of the solution. Racemisation occurs most efficiently at a pH greater than 6, without loss of solubility, which means that no acid need be added.

### Description of the Invention

For N-alkylpiperidine compounds of formula 1, the reaction is carried out in the presence of an organic cosolvent such as an alcohol or polyol, e.g. ethylene glycol thus allowing solutions of higher concentration to be used, than in the prior art. A preferred embodiment of this aspect of the invention is the racemisation of optically-enriched bupivacaine in ethylene glycol containing 10% v/v water.

The reaction conditions may comprise heating, as desired. Suitable conditions will depend on the nature of the reactants, but can be readily chosen by those skilled in the art.

In summary, the present invention establishes simple and economical processes for the racemisation of piperidine-2-carboxanilides, in aqueous media combined with inert organic cosolvents. The invention is particularly suited to the optimum utilisation of unwanted enantiomer in the preparation of enantiopure therapeutic agents, and therefore in practice the starting material will usually be richer in the (*R*)-enantiomer. When R¹ is n-butyl, the present invention is of particular utility for preparing (*S*)-bupivacaine, in conjunction with a resolution process, e.g. that described in PCT/GB95/02513 and South African Application No. 95/8993.

The following Examples illustrate the invention.

### Example 1

A mixture of (*S*)-bupivacaine (>99% ee, 1.5 g, 5.22 mmol), ethylene glycol (13.5 ml) and water (1.5 ml) was heated at 138°C for 9 hours. On cooling to ambient temperature crystallisation of a solid occurred. The solid was filtered to give a quantitative yield of bupivacaine which was shown by chiral HPLC analysis to be a 52:48 mixture of (*S*)-bupivacaine and (*R*)-bupivacaine.

### Example 2

(*S*)-Bupivacaine (>99% ee, 0.27 g, 0.94 mmol) and (*S*)-bupivacaine (-)-tartrate (2:1 salt, 0.23 g, 0.32 mmol) were heated at 150 °C in propan-2-ol (2.5 ml) and water (2.5 ml) in a sealed vessel for 22 hours. A portion of solution was removed, basified with 28% aqueous ammonia and extracted into heptane. The organic solution was dried with magnesium sulphate and the solvent removed under reduced pressure. The residue was shown by chiral HPLC to be a 63:37 mixture of (*S*)-bupivacaine and (*R*)-bupivacaine.

## Claims

1. A process for the racemisation of an optically-enriched compound of the formula 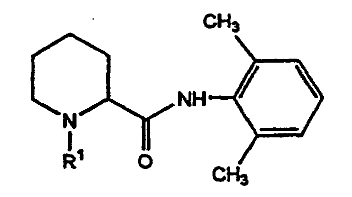 wherein R¹ is C₁₋₆ alkyl, the compound being in free base form, which comprises heating the compound in an aqueous medium which includes an organic cosolvent.

2. A process according to claim 1, wherein said compound is enriched in the (*R*)-enantiomer.

3. A process according to claim 1 or claim 2, wherein the cosolvent is an alcohol or polyol.

4. A process according to claim 3, wherein the cosolvent is an ethylene glycol.

5. A process according to any preceding claim, wherein R¹ is n-butyl, for preparing bupivacaine of diminished optical purity.

6. A process according to any of claims 1 to 4, wherein R¹ is n-propyl.

7. A process for preparing (*S*)-bupivacaine, which comprises resolving a mixture of enantiomers of bupivacaine, separating (*S*)-bupivacaine, and racemising residual (*R*)-bupivacaine by a process according to claim 5, prior to further resolution.

## Patentansprüche

1. Verfahren zur Racemisierung einer optisch angereicherten Verbindung der Formel 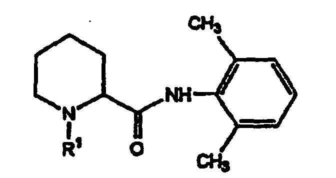 worin R¹ C₁₋₆-Alkyl ist, wobei die Verbindung in der freien Basenform vorliegt, welches das Erwärmen der Verbindung in einem wässrigen Medium, das ein organisches Co-Lösungsmittel beinhaltet, umfasst.

2. Verfahren nach Anspruch 1, worin die Verbindung bezüglich des (R)-Enantiomers angereichert ist.

3. Verfahren nach Anspruch 1 oder Anspruch 2, worin das Co-Lösungsmittel ein Alkohol oder ein Polyol ist.

4. Verfahren nach Anspruch 3, worin das Co-Lösungsmittel ein Ethylenglycol ist.

5. Verfahren nach irgendeinem vorhergehenden Anspruch, worin R¹ n-Butyl ist, zur Herstellung von Bupivacain von verminderter optischer Reinheit.

6. Verfahren nach irgendeinem der Ansprüche 1 bis 4, worin R¹ n-Propyl ist.

7. Verfahren zur Herstellung von (S)-Bupivacain, welches die Racematspaltung einer Mischung von Enantiomeren von Bupivacain, das Abtrennen von (S)-Bupivacain und das Racemisieren von restlichem (R)-Bupivacain durch ein Verfahren nach Anspruch 5 vor der weiteren Racematspaltung umfasst.

## Revendications

1. Procédé pour la racémisation d'un composé optiquement enrichi de formule 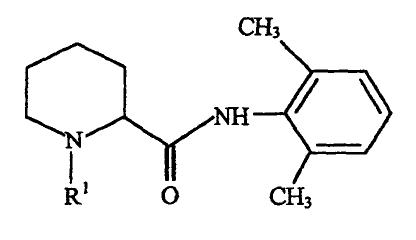 dans laquelle R¹ est un alkyle en C₁₋₆ le composé étant sous la forme de base libre, lequel procédé comprend de chauffer le composé dans un milieu aqueux qui comprend un co-solvant organique.

2. Procédé selon la revendication 1, dans lequel ledit composé est enrichi en énantiomère (R).

3. Procédé selon la revendication 1 ou la revendication 2, dans lequel le co-solvant est un alcool ou un polyol.

4. Procédé selon la revendication 3, dans lequel le co-solvant est un éthylèneglycol.

5. Procédé selon l'une quelconque des revendications précédentes, dans lequel R¹ est un groupe n-butyle, pour préparer de la bupivacaine de pureté optique amoindrie.

6. Procédé selon l'une quelconque des revendications 1 à 4, dans lequel R¹ est un groupe n-propyle.

7. Procédé pour préparer de la (S)-bupivacaïne, lequel comprend de résoudre un mélange des énantiomères de la bupivacaïne, de séparer la (S)-bupivacaïne et de racémiser la (R)-bupivacaïne résiduelle au moyen d'un procédé selon la revendication 5 avant une résolution supplémentaire.
